# EUROPEAN PATENT SPECIFICATION

(11) **EP 2 594 192 B1**
(45) Date of publication and mention of the grant of the patent: **09.09.2020**
(21) Application number: 11189197.4
(22) Date of filing: 15.11.2011
(51) Int. Cl.: A61B 3/107, A61B 3/00, A61B 3/08

(54) **Apparatus and method for analyzing ocular data**
Vorrichtung und Verfahren zum Analysieren von Augendaten
Appareil et procédé d'analyse de données oculaires

(43) Date of publication of application: 22.05.2013
(73) Proprietor: Technolas Perfect Vision GmbH, 80992 München (DE)
(72) Inventor: Gatinel, Damien, Dr., 75006 Paris (FR); Saad, Alain, Dr., 75015 Paris (FR); Youssefi, Gerhard, Dr., 84028 Landshut (DE); Zhang, Hongwei, 80997 München (DE)
(74) Representative: Vossius & Partner Patentanwälte Rechtsanwälte mbB

(56) References cited:
- US-B1- 7 110 582
- R. A. LEVINE: "Asymmetries and Visual Field Summaries as Predictors of Glaucoma in the Ocular Hypertension Treatment Study", INVESTIGATIVE OPHTHALMOLOGY & VISUAL SCIENCE, vol. 47, no. 9, 1 September 2006 (2006-09-01), pages 3896-3903, XP55023989, ISSN: 0146-0404, DOI: 10.1167/iovs.05-0469
- Donald L Budenz: "SYMMETRY BETWEEN THE RIGHT AND LEFT EYES OF THE NORMAL RETINAL NERVE FIBER LAYER MEASURED WITH OPTICAL COHERENCE TOMOGRAPHY (AN AOS THESIS)", Trans Am Ophthalmol Soc, vol. 106 1 January 2008 (2008-01-01), pages 252-275, XP002673495, Retrieved from the Internet: URL:http://www.ncbi.nlm.nih.gov/pmc/articl es/PMC2646446/ [retrieved on 2012-04-05]
- Sullivan-Mee ET AL: "Relationship Between Asymmetric Central Corneal Thickness and Glaucomatous Visual Field Loss Within the Same Patient", Optometry and vision science, vol. 83, no. 7 1 July 2006 (2006-07-01), pages 516-519, XP55024006, Retrieved from the Internet: URL:http://journals.lww.com/optvissci/Full text/2006/07000/High_Interocular_Corneal_S ymmetry_in_Average.21.aspx [retrieved on 2012-04-05]
- ALAIN SAAD, DAMIEN GATINEL: "Bilateral Corneal Ectasia after Laser in situ Keratomileusis in Patient with Isolated Difference in Central Corneal Thickness between Eyes", J. CATARACT REFRACT SURG., vol. 36, 2010, pages 1033-1035,
- RUI HUA WEI ET AL.: "Evaluation of Orbscan II Corneal Topography Individuals with Myopia", OPHTHALMOLOGY, vol. 113, no. 2, February 2006 (2006-02), pages 177-183,
- KHACHIKIAN S. STEHPEN, BELIN MICHAEL W., CIOLINO JOSEPH B.: "ntrasubject corneal thickness asymmetry", J REFRACT SURG, vol. 24, no. 6, 2008, pages 606-9,
- WEI R H ET AL: "Evaluation of Orbscan II Corneal Topography in Individuals with Myopia", OPHTHALMOLOGY, J. B. LIPPINCOTT CO., PHILADELPHIA, PA, US, vol. 113, no. 2, 1 February 2006 (2006-02-01), pages 177-183, XP027899909, ISSN: 0161-6420 [retrieved on 2006-02-01]

## Description

### BACKGROUND OF THE INVENTION

### 1. Field of the Invention

The present invention relates to an apparatus and a method for analyzing ocular data, preferably corneal data, in particular to a corneal tomography analysis system and a method of performing corneal tomography analysis.

### 2. Description of the Related Art

Corneal tomography provides 3-dimensional (3-D) reconstruction of the cornea, enabling evaluation of the anterior and posterior corneal surfaces and creation of a pachymetric map. Commercially available corneal tomography systems use at least four methods: horizontal slit-scanning (Orbscan II®, Bausch & Lomb), rotating Scheimpflug camera (Pentacam®, Oculus), very-high-frequency ultrasound (Artemis®, Ultralink), and high-speed anterior segment optical coherence tomography (Visante®, Zeiss). The corneal tomography is displayed in terms of various kinds of color maps.

Corneal shape corrective surgeries (refractive surgeries) are commonly used to treat myopia, hyperopia, astigmatism, and the like. Procedures employing an excimer laser include laser assisted in-situ keratomileusis (LASIK), photo refractive keratectomy (PRK) and laser sub-epithelial keratomileusis (LASEK). Corneal Ectasia is an eye disease and is one known complication of refractive surgery. There has been a great interest in attempting to identify preoperatively patients at risk for this complication. It's now known that corneas that share similarities with Keratoconus (KC) or Pellucid Marginal Corneal Degeneration (PMD) are at higher risk for this complication. So the major goal in preventing post Lasik ectasia is to detect KC and PMD in their earliest and mildest stage.

The information about the corneal tomography obtained by such an analysis system can be utilized for planning a refractive surgery. In addition, the corneal tomography is used for early discovery and diagnosis of corneas susceptible to develop corneal ectasia post-operatively.

As an early diagnosis of keratoconus, a method of judging keratoconus tomography using a discriminant function approach is disclosed by Alain Saad and Damien Gatinel in "Topography and Tomography Properties of Forme Fruste Keratoconus Corneas, Investigative Ophthalmology & Visual Science, November 2010, Vol. 51, No. 11, pp. 5546-5555" (Reference 1).

In this method, the presence of keratoconus (KC) and Forme Fruste Keratoconus (FFKC) are judged using a discriminant function and 34 indexes characterizing the corneal topography and tomography, i.e., Irregularity at 3mm, Irregularity at 5mm, Thickness of the Thinnest Corneal Point (TP), Thickness of the Corneal Apex (CP), Difference in Thickness between the TP and the CP, Maximum Anterior Elevation in a 1mm radius centered on the Corneal Apex as compared to a Best Fit Sphere (MAE), Maximum Posterior Elevation in a 1mm radius centered on the Corneal Apex as compared to a Best Fit Sphere (MPE), Anterior Elevation of the Thinnest Point (AETP), Posterior Elevation of the Thinnest Point (PETP) and the Anterior, Posterior and pachymteric spatial profile. The topographic and tomographic studied items and the display of the results of the analysis leave room for further improvement.

In the Article "Bilateral Corneal Ectasia after Laser in situ Keratomileusis in Patient with Isolated Difference in Central Corneal Thickness between Eyes" of Alain Saad and Damien Gatinel, J. Cataract Refract Surg. 2010; 36:1033-1035 (Reference 2) it is disclosed that the central thickness difference between the right eye and the left eye of a patient could correspond to an earliest manifestation of a pathologic cornea. This observation shall suggest that a topographic asymmetry or difference in central pachymetry between eyes of a patient should be considered an additional risk factor for developing post-LASIK ectasia. Stepen S. Khachikian et al. "Intrasubject Corneal Thickness Asymmetry", J Refract Surg 2008; 24(6): 606-609 (Reference 3) discloses that the pachymetry asymmetry between both eyes of a patient which is outside a normal range should learn a clinician to examine for other parameters that are more established refractive surgery risk factors. Right and left eye pachymetry values were compared for the corneal apex, pupil center and thinnest point. A statistical analysis was performed to determine normal levels of variants. The authors found that an average pachymetry difference between fellow eyes at the corneal apex was 8.8 ±7.2 µm. The average pachymetry difference between fellow eyes at the pupil center was 8.9 ± 8.3 µm. The average pachymetry difference between fellow eyes at the thinnest point was 9.0 ± 8.3 µm.

In the Article "Neural Network Classification of Corneal Topoghrapy" of Naoyuki Maeda et al. Invest Ophtalmol Vis Sci 1995; 36(7): 1327-1335 (Reference 4) it is suggested to evaluate topographic abnormalities of the cornea by using a neural network classification system. Corneal topographic maps were obtained from a patient population with a computer assisted videokeratoscope. The maps were randomly divided into a training set and a test set. Eleven quantitative descriptors from each map were used as input data for a neural network model. These topographic indices were selected and devised to extract the characteristics of corneal topographic patterns. The subjects in each set, i.e. the training set and the test set comprise normal cornea, with - the - rule astigmatism, keratoconus, mild, moderate and advanced, postphotorefractive keratectomy and postkeratoplasty. In this neural network system the input layer received the topographic indices as input data and the output layer outputs output data corresponding to the before-mentioned categories of the different types of subjects in each set. The hidden layer in between the input layer and the output layer was trained with a respective data from the sets. More specifically, each of the input values is multiplied by a corresponding weight and the sum of the weighted inputs is transformed by a non-linear transfer function. Several layers are present in this neural network system. Neural networks shall offer advantages over other methods for a computer-assisted diagnosis such as multivariate analysis using a discriminant analysis or a conventional artificial intelligence approach using an expert system.

Richard A. Levine et al., "Asymmetries and Visual Field Summaries as Predictors of Glaucoma in the Ocular Hypertension Treatment Study" invest. Ophtalmalogy and Visual Science, September 2006, Vol. 47(9): 3896-3903 (Reference 5) evaluate whether baseline visual field data and asymmetries between eyes predict the onset of primary open angle glaucoma (POAG) in Ocular Hypertension Study (OHTS) participants. In this study a new index, a mean prognosis (MP), was designed for optimal, combination of visual field thresholds, to discriminate between eyes that developed POAG from eyes that did not. The baseline intraocular pressure (IOP) in fellow eyes was used to construct measures of IOP asymmetry. Age-adjusted baseline thresholds were used to develop indicators of visual field asymmetry and summary measures of visual field defects. Marginal multivariate failure time models were constructed that they relate the new index MP, IOP asymmetry, and visual field asymmetry to POAG onset for OHTS participants.

The Article "Mapping Collagen Organization in the Human Cornea: Left and Right Eyes are Structurally Distinct" of Craig Boote et al., Investigative Ophthalmology & Visual Science, March 2006, Vol. 47(3): 901-908 (Reference 6) the organization of collagen fibrils and the corneal stromal of left and right eyes is examined and compared. It is stated that aspects of the biomechanics and surface topography of fellow human corneas are known to exhibit midline symmetry, but the structural basis of these observation is purely understood. It is also stated that the surface topography of fellow normal human corneas exhibits a degree of non-superimposable midline symmetry. It is suspected that the precise organization of stromal collagen has a role to play in determining corneal shape through a direct impact on tissue biomechanics. Intrinsic structural features may predispose fellow corneas to the kind of enantiomorphic ectasia reported in keratoconus. Therefore, computational models of corneal biomechanics also incorporate fibril orientation data, with the purpose being to simulate more effectively the tissues response to surgery.

Stephen D. Klyce "Chasing the Suspect: Keratoconus" British Journal Ophtalomolgy 2009 93: 845-847 (Reference 7) discusses several proposals to detect keratoconus suspect and forme fruste keratoconus. With reference to one of these studies it is stated that just over 20% of the fellow eyes progressed to clinical keratoconus with evidence of increased asymmetry, spherical equivalent and higher-order irregularities, which have been documented with topographic indices and Fourier analysis. According to this study the asymmetry of a single cornea is analyzed for detecting such irregularities. As a possible treatment to manage ectasia collagen cross-linking is suggested.

Rui Hua Wei et al. "Evaluation of Orbscan II Corneal Topography Individuals with Myopia", Ophthalmology Vol. 113, No. 2, February 2006, pages 177-183 (Reference 8) discloses a study of evaluating topography in individuals with myopia. In this study, the Orbscan II corneal topography system now Orbscan II® (Bausch & Lomb, Orbtek Inc., Salt Lake City, UT) is used to detect abnormal cornea shapes, such as Keratoconus and contact lens-induced cornea! thinning. To detect early abnormal changes in corneal morphologic features, a data base of topography in normal human eyes is used to established baseline indices. With reference to previous studies of videos of your keratography patterns and quantitative indices of normal human corneas it is stated that a high degree of mirror image symmetry between fellow eyes has been found. The intraocular corneal symmetry in average simulated keratometry, central corneal thickness, and posterior elevation at the point of fixation were assessed by comparative data analysis. In this study the symmetry in corneal elevation maps and pachymetry maps between right and left eyes in each subject based on both indices and patterns was evaluated and the variations in the corneal topographies were assessed as a function of maximum corneal curvature. Topographic maps of 140 eyes in 70 subjects with myopia who enrolled for preoperative assessment for LASIK were reviewed retrospectively. Subjects having any type of ocular pathologic features or surgery or family history of keratoconus were not included in this study. By using the Orbscan II corneal topography system 20 separate elevation maps were obtained. The instruments software analyzed 9600 points on the entire cornea and calculated the anterior and posterior corneal elevations (relative to a best-fit sphere) the axial curvature map of anterior corneal surface, the corneal thickness, the cornea and pupil sizes, and anterior chamber depth. All data, including demographic information, manifest refraction, and quantitative data from each Orbscan II corneal topography, were entering to a data base. The Orbscan II corneal topography system produces several quantitative indices including
1. Anterior elevation best fit sphere (BFS; diopters)
2. Posterior elevation BFS (diopters)
3. Maximum posterior elevation (Max PE; micrometers), theoretically determined by taking the maximum difference in the posterior elevation between the BFS and the patient's cornea.
4. Radius of the Max PE (millimeters): the distance from the apex of the cornea (highest elevation with respect to the camera).
5. Maximum simulated keratometry (Max K; diopters).
6. Minimum simulated kertometry (Min K; diopters).
7. Astigmatism (Astig; diopters): difference between Max K and Min K.
8. Irregularity (Irreg.), mean power, and astigmatism of the anterior corneal surfaces in the 3-mm and 5-mm diameter zones (diopters).
9. Corneal diameter (white-to-white [WTW], millimeters).
10. Pupil diameter (millimeters).
11. Thinnest pachymetry (micrometers): the minimum difference in elevation between anterior and posterior corneal surfaces. An acoustic equivalent correction factor of 0.92, as recommended by the manufacturer, was applied to calculate corneal thickness values.
12. Anterior chamber depth (ACD; millimeters).

During a statistical analysis the measurement from each eye was evaluated. Correlations of indices between the right and left eyes and correlation between each Orbscan II and maximum keratometry were explored using the Pearson's correlation coefficient (r). The correlation between two eyes showed a mirror-imaged symmetry between right and left eyes in 77% of anterior elevation topographs, 73% of posterior elevation topographs, and 64% of pachymetry topographs. Given that corneal topographies for right and left eyes are correlated, the right eyes of the 70 subjects were used to assess the correlation between each Orbscan II and maximum keratometry. The authors of this Article concluded that there is a significant mirror image symmetry between the right and the left eyes. Based on this conclusion the authors recommend that studies analyzing and comparing the control population should use only one eye of each normal subject. Using both eyes of each subject would result in overestimation or underestimation of indices. In this Article, further reference is made to the study evaluating eyes of patients with keratoconus. In this further study it was found that the apex elevation of eyes with keratoconus correlated well with apex composite curvature and apex tangential curvature. In another study it was shown that eyes with positive keratoconus screening criteria had higher posterior elevation on Orbscan II topography. It was recommended that patients with a positive keratoconus screening using Tomey topography and a posterior elevation of more than 40 µm be identified as having a high risk of ectasia after LASIK. On the other hand, in the present study it was found that maximum posterior elevation did not correlate with corneal curvature. This shall suggest that posterior cornea! elevation will not vary with the degree of corneal curvature in a normal subject.

US 7 370 969 B2 relates to a corneal topography analysis system which comprises an input unit for inputting curvature data and an analysis unit that determines plural indices characterizing topography of the cornea based on the input corneal curvature data. The analysis unit further judges corneal topography from feature inherent in predetermined classifications of corneal topography using the determined indexes and a neural network so as to judge at least one of normal cornea, myopic refractive surgery, hyperopic refractive surgery, corneal astigmatism, penetrating keratoplasty, keratoconus, keratoconus suspect, pellucid marginal degeneration, or other classification of corneal topography.

### SUMMARY OF THE INVENTION

It is an object of the present invention to provide an apparatus and a method for analyzing ocular data, preferably corneal data. This object is solved with the features of the independent claims.

One aspect of the present invention relates to an apparatus and a method for analyzing ocular data that allow the detection of an ocular disease, for example keratoconus, glaucoma or Marfan disease. Marfan disease is an inherited dystrophy of elastic tissue with ophthalmic expression, for example a crystalline lens deformation and luxation, which is usually asymmetric between a right eye and a left eye of a patient. This detection is based on analyzing asymmetries of ocular data between the two eyes of a patient.

The ocular data may comprise data obtained by measurements of the topography and/or biomechanical characteristics of the eye. In particular, corneal hysteresis and/or corneal resistance factor may be evaluated, which may be obtained for example with an Ocular Response Analyzer. The ocular data may also comprise data obtained by dynamic ultra high-speed Scheimpflug technology non-contact tonometry (for example with a Corvis ST, Oculus, Germany). The comparison of the corneal deformation process under a symmetrically metered air pulse can provide biomechanical assessment and comparison between the two eyes of a patient.

One further aspect of the present invention relates to a corneal tomography analysis system, and a method of performing corneal tomography analysis that allow the detection of ectasia susceptible corneas more precisely.

Traditional screening strategies are based on artificial intelligence and neural network focusing on detecting keratoconus suspect (KCS) by analyzing various indices of corneal shape characteristics but it all considers each eye as an independent entity. According to the present invention both eyes of an individual are evaluated based on the realization that both eyes of the same individual have the same genetic makeup. It has already been proven that an asymmetry in ocular parameter such as intra-ocular pressure or cup/disc ratio is a predictor of pathology. Furthermore, a few studies showed that the surface topography and the higher-order ocular optical aberrations of fellow normal human corneas exhibit a degree of midline symmetry, whereas the keratometry readings, the central corneal thickness and the posterior elevation present a high interocular symmetry. The inventors found that an asymmetry in the spatial profile between both eyes may be an early manifestation of a pathological process affecting the cornea.

According to one other aspect of the invention the input unit is configured to receive ocular data from at least one of a plurality of same-in-class measuring devices and the input unit is preferably configured to transform ocular data from said at least one plurality of same-in-class measuring devices into one common data format which is used for analyzing said ocular data. The same-in-class measuring devices preferably comprise corneal topographic measuring devices and/or tomographic measuring devices

The methods for the acquisition of the 3D reconstruction of the cornea are different among corneal tomography systems of different manufacturers. Furthermore, the number of data items and the data structure are different among the manufacturers of the systems. Generally, therefore, there is no data comparability among the manufacturers. Therefore, according to a further aspect of the present invention corneal tomography is analyzed based on point data for the data structure for each individual corneal tomography systems of a manufacturer.

According to another aspect the present invention provides a corneal topographic and tomography analysis system comprising an input unit, preferably one that can receive input from different corneal topographic and tomographic measuring devices, for entering anterior, posterior and pachymetric spatial profile data. The system further comprises an analysis unit for determining plural indexes characterizing topography and/or tomography of each studied cornea based on the input corneal data, for comparing the right eye indexes to the left eye indexes of the same patient, preferably determining an Enantiomorphism Index. The system further comprises means for judging corneal topography and/or tomography from features inherent in predetermined classifications of corneal topography and/or tomography using the determined indexes and the Enantiomorphism Index (EI). The system further comprises means for judging at least one of normal cornea or ectasia susceptible cornea and for judging its probability. In addition, the system comprises a display unit for displaying results of the judgments made by the analysis unit.

Other objectives and features of the invention, including methods of carrying out the invention, are described hereunder by way of examples and with reference to the drawings.

### BRIEF DESCRIPTION OF THE DRAWINGS

- Fig. 1: shows a flow diagram of a first embodiment according to the present invention.
- Fig. 2: shows a flow diagram of a comparative example,
- Fig. 3: shows a diagram of a first example to combine right eye data and left eye data according to the present invention,
- Fig. 4: shows an example of a tomography map from the right eye and the left eye of a patient before and after transformation of the data of the right eye,

- Fig. 5: shows on the left side an example of the pachymetry data of the right eye and the left eye of a patient and the resulting difference after subtracting pachymetry data of the left eye from the pachymetry data of the right eye,
- Fig. 6: shows in the upper part a schematic diagram of the curvature map of the right eye and the curvature map of the left eye of a patient, wherein the curvature map of the right eye is shown before and after transformation and shows in the lower part the resulting difference by subtracting the curvature map of the right eye from the transformed curvature map of the left eye.
- Fig. 7: shows an example for determining combined indices from preceding right eye indices and left eye indices, and
- Fig. 8: shows a block diagram of a system for acquiring, analyzing and displaying corneal data.

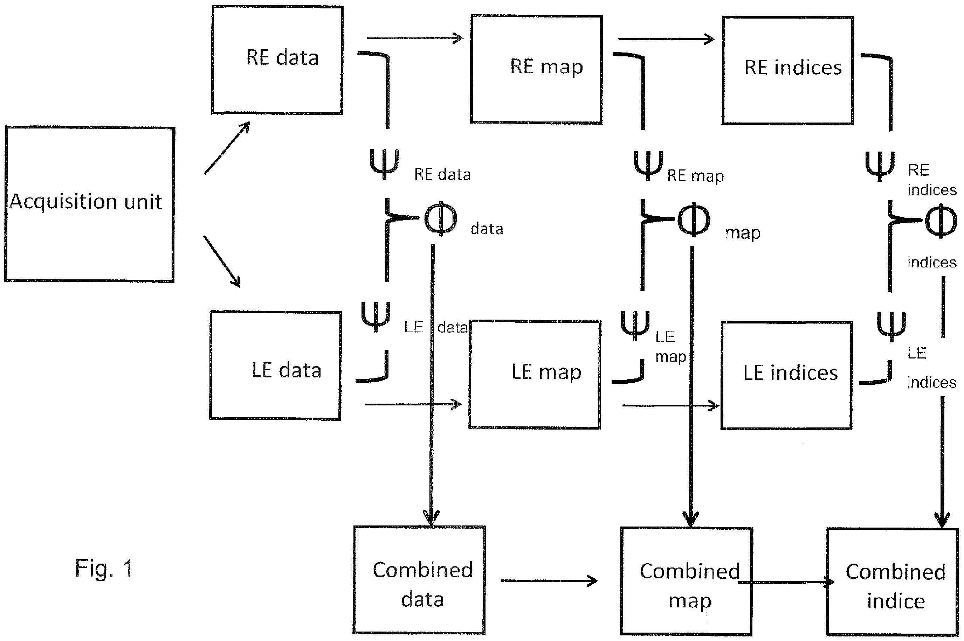

According to a first embodiment of the present invention corneal data as an example of ocular data of the right eye and corneal data as an example of ocular data of the left eye of the patient are analyzed as shown in the flow diagram of Fig. 1. More specifically, an acquisition unit is used for obtaining and providing corneal data of the right eye and corneal data of the left eye of a patient. These corneal data may comprise raw data of the right eye, designated in Figure 1 as RE data and may comprise raw data of the left eye, designated in Figure 1 as LE data. These raw data can be further processed to obtain for example the elevation information and/or the curvature topography information related to each eye separately. The raw data of the right eye and the raw data of the left eye can be further processed into various maps and indices. For example, as shown in Fig. 1 the raw data are first processed into a respective map of the right eye, designated in Figure 1 as RE map and a map of the left eye, designated in Figure 1 as LE map. As further indicated in Fig. 1 the right eye map can be further processed to obtain indices of the right eye, designated in Figure 1 as RE indices and the left eye map can be further processed into indices of the left eye, also designated in Figure 1 as LE indices.

According to the preferred embodiment of the invention the raw data of the right eye are transformed into transformed data. In addition the raw data of the left eye are transformed into respective transformed data of the left eye. Thereafter the transformed data of the right eye and the transformed data of the left eye are combined to obtain combined data. In Fig. 1 each transformation is indicated as transformation Ψ_{REdata} and Ψ_{LEdata}, respectively and the combination is indicated as combination Φ_{data}. Alternatively, raw data of the right eye are combined with transformed data of the left eye. As a further alternative, the raw data of the right eye and the transformed data of the left eye are combined.

Each of the before-mentioned transformations Ψ_{REdata} and Ψ_{LEdata} may comprise one or several of the following operations, a) an inversion of raw data, preferably with respect to a vertical axis parallel to a nasal line between the two eyes of a patient, b) an operation to achieve symmetry between corneal data of the right eye and corneal data of the left eye, c) a translation of data, for example along a horizontal and/or vertical axis in a cartesian coordinate system, d) a shift of data along the horizontal axis and/or the vertical axis of said cartesian coordinate system and e) a rotation of data around a reference point of the right eye and/or the left eye.

Each of the before-mentioned combinations of Φ_{data} may comprise at least one of the following operations, a) a subtraction of corneal data of the right eye from the corneal data of the left eye wherein either or both corneal data of the right eye and the left eye have undergone transformation, b) an intersection of corneal data of the right eye and corneal data of the left eye and c) a summation of corneal data of the right eye and corneal data of the left eye.

In addition, the map of the right eye and/or the map of the left eye is transformed and then combined φₘₐₚ into a combined map. The transformation ψ_{Remap}, ψ_{LEmap} of the right eye map and/or the left eye map comprises at least one of the following operations, an inversion of the map, an operation for providing symmetry of the right eye map and the left eye map, a translation of the map, a shift of the map and a rotation of the map.

As a further optional feature the right eye indices and the left eye indices are transformed ψ_{REindices}, ψ_{LEindices} and combined indices after one or both of the indices are transformed. From each of the combined data, and/or a combined map and the directed combination of right eye indices and left eye indices combined indices are generated.

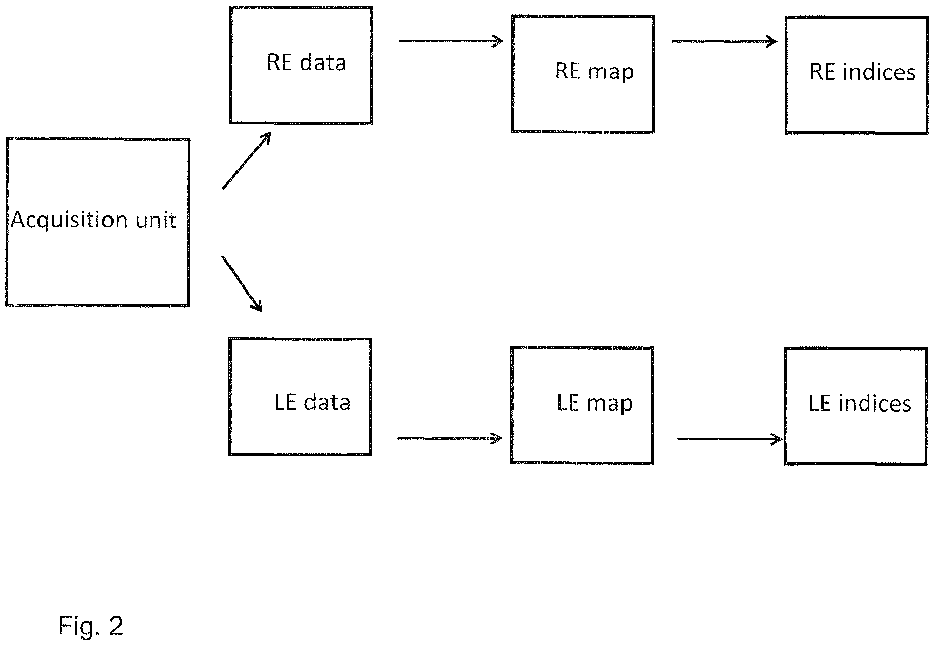

A comparative example of a flow diagram is shown in Fig. 2. According to this comparative example an acquisition unit obtains and provides corneal data. In this example raw data of the right eye designated as RE_{data} of the left eye, and raw data of the left eye, designated as LE_{data} are provided. The raw data of the right eye RE_{data} are further processed to obtain a right eye map REₘₐₚ. This right eye map is further processed to obtain indices of the right eye designated as RE indices. Similarly, the raw data of the left eye are first processed into a respective map of the left eye designated as LEₘₐₚ and the left eye map is further processed into indices of the left eye designated as LE indices. According to this exemplarily embodiment conventional elevation and curvature topography provides information related to each eye separately. This information is derived from the raw data, for example RE_{data} which is processed into various maps and indices. Different from the embodiment as shown and described with reference to Fig. 1 there are no combinations of raw data of the right eye and the left eye or combinations of the derived map of the right eye and the left eye or a combination of the indices of the right eye and the left eye.

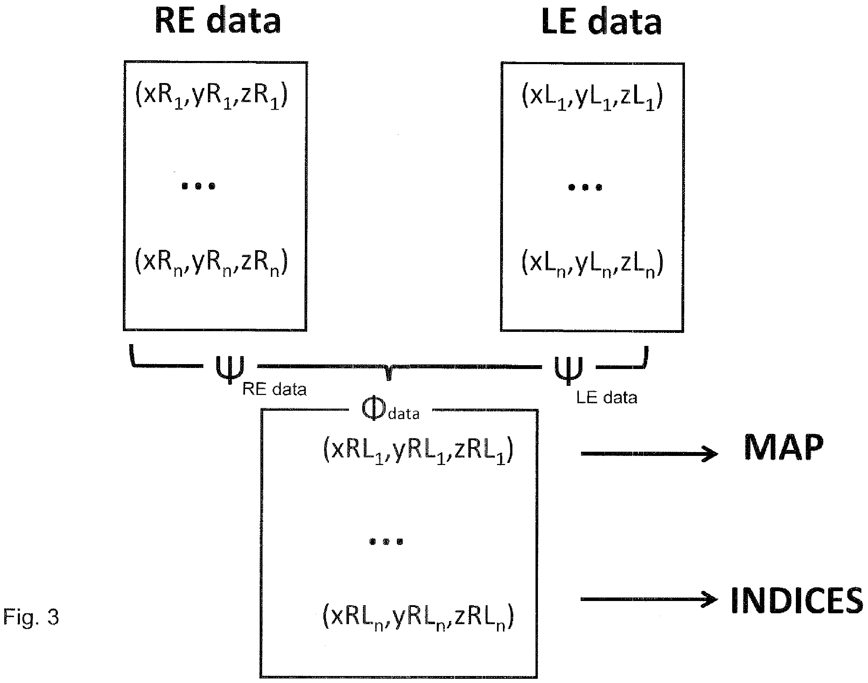

In Fig. 3 a first example to combine right eye data and left eye data according to the present invention is shown in form of a diagram. More specifically, the raw data of the right eye RE_{data} and the raw data of the left eye LE_{data} are each provided by a matrix box. In this example, each matrix, i.e. the matrix for the right eye data and the matrix for the left eye data comprise the same number n of data sets. Each data set comprises three different data in the form of triplets (xR₁), (yR₁), (zR₁), ...(xRₙ, yRₙ, zRₙ) which correspond to the coordinate of a point of the cornea surface of interest of the right eye. Similarly, each data set of the left eye comprises three data in the form of triplets (xL₁, yL₁, zL₁) ...(xLₙ, yLₙ, zLₙ) which correspond to the coordinate of a respective point of the cornea surface of interest of the left eye. In the example, as shown in Fig. 3 the data of each matrix box are transformed by using a respective transformation ψ_{REdata} for processing the data of the right eye and a transformation ψ_{LEdata} for the left eye. After transformation the transformed data are combined by using a combination φ_{data}. These combination data are represented by a further matrix box which comprises n data sets. Each of these data sets comprises data in the form of triplets (xRL₁, yRL₁, zRL₁) ... (xRLₙ, yRLₙ, zRLₙ). Starting out from this matrix box which is a result of the combination further maps or indices can be computed. This is diagrammatically shown as an arrow leading to the word map and an arrow leading to "indices".

According to a specific example the left matrix box 1 comprises raw elevation data of the right eye and the right matrix box comprises raw elevation data of the left eye of a patient. In other words, for each horizontal position which is described with a respective value along the X-axis and along the Y-axis the respective elevation designated as z is given for a particular data set. According to an example the raw elevation data from the right eye could be inverted horizitonally and shifted in the direction of the X-axis or the Y-axis or rotationally shifted with respect to a reference point until the data from the right eye reaches a best fit of the elevation data of the left eye. This transformation ψ_{REdata} may be performed solely for the raw data of the right eye or alternatively solely for the raw data of the left eye or as shown in Fig. 3 this transformation can be performed with respect to the raw data of the right eye and the raw data of the left eye. Once the distance between the two set of points is minimized, a map showing the residuals, calculated as a difference in elevation between the respective surface points is generated. Furthermore, whereas indices characterizing the level of enantiomorphism is computed. For example, one calculated index is the RMS value of the residuals.

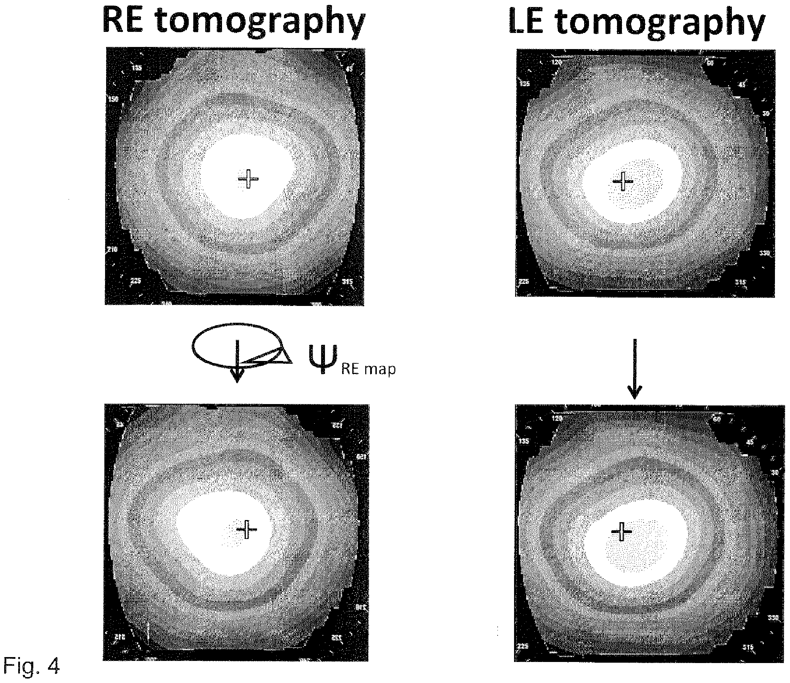

In Fig. 4 tomography maps obtained for the right eye and for the left eye are shown. The thinnest point location of the corneal wall can be identified for each eye. With respect to this thinnest point location of the corneal wall in each eye a best fit of the tomography map of the right eye and the tomography map of the left eye can be achieved. In this example, the right eye data is inverted horizontally using a transformation ψ_{Remap} by using the horizontal symmetry. The result of this transformation of the right eye tomography is shown in the lower left diagram of Fig. 4. On the other hand, the left eye tomography is not transformed in this example, i.e. the map as shown in the lower right part of Fig. 4 is the same as shown in the upper right part of Fig. 4.

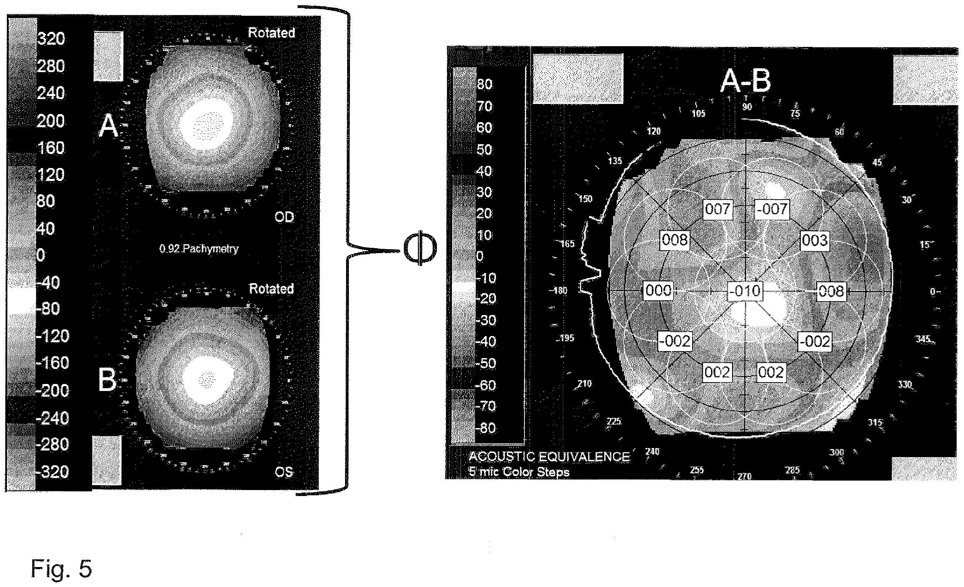

Fig. 5 shows an example of pachymetry data of the right eye and the left eye of a patient. The respective map A and B are shown in the left part of Fig. 5. The sets of corneal data of the left eye and the right eye are aligned with respect to the thinnest point location of the corneal wall which is identified for each eye. The sets of corneal data are then superimposed. More specifically, a subtraction from the centrally thicker to the thinner thickness set of data is performed by using a combination φ and displayed in the right part of Fig. 5. Thus, the resulting difference A-B is shown in this diagram. Exemplarily values indicating the difference are given as 000, -002, -007 and -010 and 002, 007 and 008.

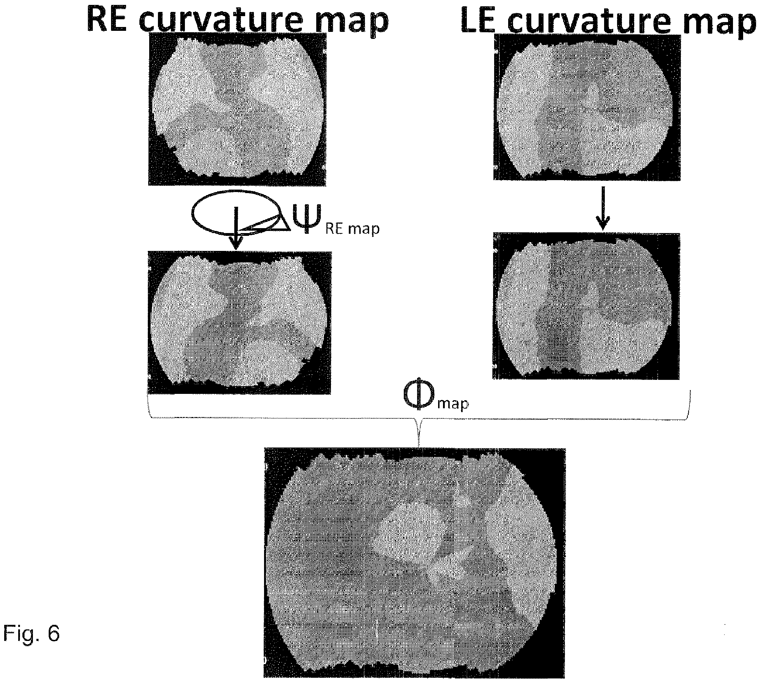

In a further example as shown in Fig. 6 the raw data is first processed to generate actual curvature maps for the right and the left eyes anterior corneal surfaces, respectively. The right eye map is inverted horizontally by using a transformation ψ_{Remap}. Then an operation is performed in which the right eye curvature data is aligned with the left eye curvature data, so that the points corresponding to the vertex of each eye are superimposed. If appropriate, additional rotation of the right eye data can be performed. Then, the subtraction of data is made between the data of the two surfaces using the combination φₘₐₚ. In the lower part of
Fig. 6 the resulting difference in actual curvature between the right and left anterior corneal surfaces is shown.

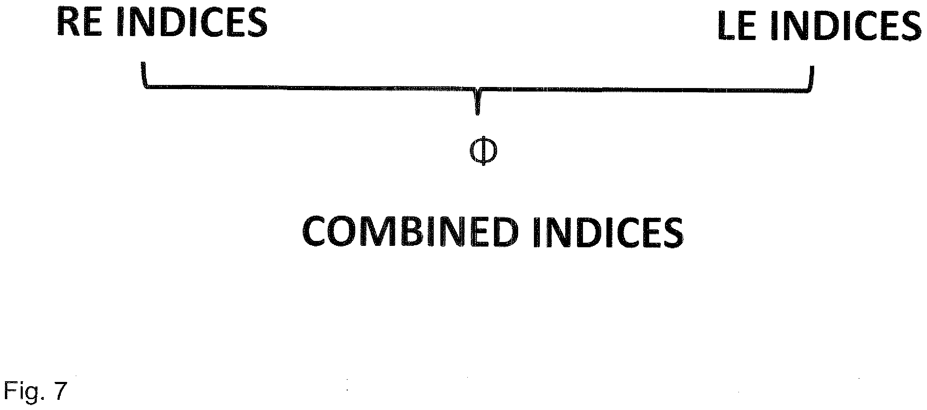

Fig. 7 shows an example how to determine combined indices by performing a calculation using the preceding indices of the right eye RE indices and the indices of the left eye LE indices.

Analysis regarding judgment of the classifications of corneal topographies is described next. On the basis of the corneal topographic and tomographic data obtained, several indexes are calculated. For example, the following indices are calculated:
1) Percentage of Decrease in Thickness (PDT, PIT in the excel sheet) from the periphery of the cornea to the Thinnest Point (TP) at different distances from the TP
2) Percentage of variation of anterior curvature (PVAK) from the TP to the periphery of the cornea at different distances from the TP.
3) Percentage of modification of posterior curvature (PVPK) from the TP to the periphery of the cornea at different distances from the TP.
4) Percentage of modification of total power (PVTP) from the TP to the periphery of the cornea at different distances from the TP.
5) Central pachymetry (CP)
6) Thinnest pachymetry (TP)
7) Maximum Anterior Elevation (MAE)
8) Maximum Posterior Elevation (MPE)
9) Anterior Elevation at the TP (AETP)
10) Posterior Elevation at the TP (PETP)
11) Anterior Best Fit Sphere (ABFS)
12) Posterior Best Fit Sphere (PBFS)
13) Anterior Peripheric Best Fit Sphere (ABFS Periph)
14) Posterior Peripheric Best Fit Sphere (PBFS Periph)
15) Anterior Elevation at the TP calculated with a peripheric BFS (AETP Periph)
16) Posterior Elevation at the TP calculated with a peripheric BFS (PETP Periph)
17) Maximum Anterior Elevation calculated with a peripheric BFS (MAE Periph)
18) Maximum Posterior Elevation calculated with a peripheric BFS (MPE Periph)
19) Irregularity at 3mm
20) Irregularity at 5mm
21) Position of the thinnest point (TP Position).
22) SIM K max
23) SIM K min
24) Sim K Difference
25) Difference between Central Pachymetry and Thinnest Pachymetry (CP-TP)

These plural indexes and the comparison of these plural indexes between the right eye and the left eye are taken as input data, and the probability (%) of the classification of each of various cornea! topographies is output using a discriminant function.

The decisions of the classifications of the corneal topographies and their probabilities can be found as described next by entering the plural indexes and the between eyes comparison of the plural indexes into the discriminant function.

By using at least some or all indices the combined indices for a patient can be compared to combined indices of a reference group of patients. The differences between the right eye and the left eye of a patient and the right eye and the left eye of a reference group can be analyzed. From this analysis the inventors found that differences between the right eye and the left eye are much higher in the case where the patient has a keratoconic eye in comparison to the reference group where the persons have normal eyes. According to further preferred embodiment of the present invention plural combined indices of a patient are compared with corresponding plural combined indices of a reference group. A comparing unit can be used which makes use of at least one discriminant function which comprises input variables and respective coefficients. The coefficients are based on a statistical analysis of corneal data of a plurality of patients of a reference group. The plural combined indices of a patient are taken as input data for the corresponding input variables of the discriminant functions. Optionally, the further plural combined indices obtained from the combined map are also taken as input data for the corresponding input variables of a respective discriminant function. In addition, other plural combined indices are taken as input data for the corresponding input variables of a respective discriminant function which are obtained as combined indices from right eye indices and left eye indices.

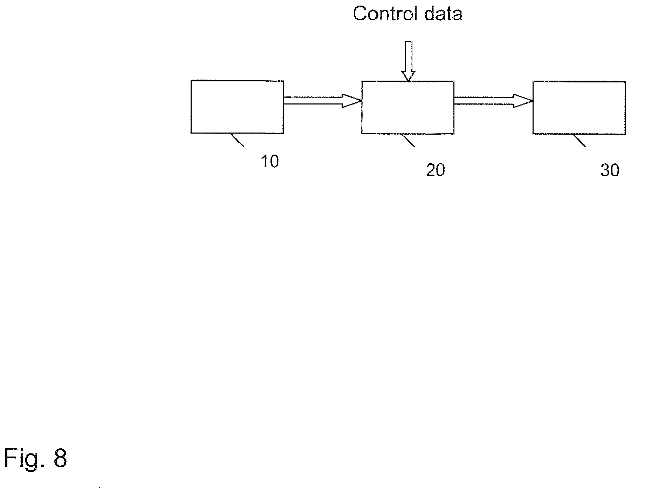

In Fig. 8 an example of a system for acquiring, analyzing and displaying corneal data is shown. More specifically, an acquisition unit 10, for example a topography or a tomography system obtains data from a patient, in particular corneal data of the right eye and the left eye of a patient. These data are provided to an apparatus for analyzing the corneal data 20. The analyzing unit further receives control data, for example the predetermined classifications of corneal tomography and/or of corneal topography. The analyzing unit 20 is configured to perform the analysis and shown and described with reference to Figs. 1-7. In this embodiment the result is provided to a display unit 30.

More specifically, the acquisition unit provides corneal data preferably anterior elevation data, posterior elevation data and pachymetry spatial profile data. The analyzing apparatus comprises an input unit for receiving corneal data of the right eye and the corneal data of the left eye of a patient. It further comprises a combining unit receiving the corneal data of the right eye and the corneal data of the left eye for generating combined data for the patient. The analyzing apparatus further comprises an analysis unit receiving the combined data for determining plural combined indices. These plural combined indices are used for diagnosis of corneal diseases by using control data derived from a population group.

The display unit is configured to receive and display any of the data received and processed in the apparatus for analyzing corneal data.

The present invention has now been described with reference to several embodiments thereof. Special reference has been made to analyzing corneal data. The present invention is generally related to the analysis of ocular data that allow the detection of an ocular disease. The foregoing detailed description and examples have been given for clarity of understanding only. No unnecessary limitations are to be understood therefore. It will be apparent to those skilled in the art that many changes can be made in the embodiments described without departing from the scope of the present invention, as defined in the appended claims.

References:
(1) Saad Alain, Gatinel Damien. Topography and Tomography Properties of Forme Fruste Keratoconus Corneas, Investigative Ophthalmology & Visual Science, November 2010, Vol. 51, No. 11, pp. 5546-5555
(2) Saad Alain, Gatinel Damien. Bilateral corneal ectasia after laser in situ kerateumileusis in a patient with an isolated difference in central corneal thickness between both eyes. J Cataract Refract Surg 2010; 36:1033-1035
(3) Khachikian S. Stehpen, Belin Michael W., Ciolino Joseph B. Intrasubject corneal thickness asymmetry. J Refract Surg 2008;24(6):606-9.
(4) Maeda Naoyuki, Klyce Stephen D., Smolek Michael K. Neural network classification of corneal topography. Preliminary demonstration. Invest Ophthalmol Vis Sci 1995;36(7):1327-35.
(5) Levine Richard A., Demirel Shaban, Fan Juanjuan, et al. Asymmetries and visual field summaries as predictors of glaucoma in the ocular hypertension treatment study. Invest Ophthalmol Vis Sci 2006;47(9):3896-903.
(6) Boote Craig, Hayes Sally, Abahussin Mohammad, Meek Keith M. Mapping collagen organization in the human cornea: left and right eyes are structurally distinct. Invest Ophthalmol Vis Sci 2006;47(3):901-8.
(7) Stephen D. Klyce "Chasing the Suspect: Keratoconus" British Journal Ophtalomolgy 2009 93: 845-847
(8) Rui Hua Wei et al. "Evaluation of Orbscan II Corneal Topography Individuals with Myopia", Ophthalmology Vol. 113, No. 2, February 2006, pages 177-183

## Claims

1. Apparatus for analyzing corneal data comprising:
an input unit configured to receive corneal data of the right eye and corneal data of the left eye of a patient, said corneal data comprising at least one of elevation corneal data, topographic corneal data, tomographic corneal data, anterior corneal data, posterior corneal data and pachymetric spatial profile data;
a combining unit configured to receive the corneal data of the right eye and the corneal data of the left eye for generating combined data for the patient,
an analysis unit configured to receive the combined data for determining plural combined indices, wherein the analysis unit is configured to use said plural combined indices for diagnosis of corneal diseases by using control data derived from a population group.

2. Apparatus according to claim 1, further comprising:
at least one transformation unit for transformation of the corneal data of the right eye and/or the corneal data of the left eye received from said input unit, and for providing transformed data to the combining unit.

3. Apparatus according to claim 2, wherein said transformation unit is configured to perform at least one of the following operations, an inversion of data, preferably with respect to a vertical axis, an operation to achieve symmetry between corneal data of the right eye and corneal data of the left eye, a translation of data, a shift of data and a rotation of data.

4. Apparatus according to claim 2 or 3, wherein the transformation unit is adapted to perform a transformation of corneal data of the right eye and/or corneal data of the left eye to achieve a best fit of the corneal data of the right eye with the corneal data of the left eye.

5. Apparatus according to claim 4, wherein the best fit of the corneal data of the right eye with the corneal data of the left eye is achieved preferably by referring to one of the following reference criteria: a) best fit of the elevation of the corneal data of the right eye and the elevation of the corneal data of the left eye, b) that the corneal data of the right eye and the corneal data of the left eye are aligned with respect to the thinnest point of the cornea of the right eye and the thinnest point of the cornea of the left eye, c) that the corneal data of the right eye and the corneal data of the left eye are aligned with respect to the corneal vertex of the cornea of the right eye and the corneal vertex of the cornea of the left eye, d) that the corneal data of the right eye and the corneal data of the left eye are aligned with respect to the corneal apex of the cornea of the right eye and the corneal apex of the cornea of the left eye, and e) that the corneal data of the right eye and the corneal data of the left eye are brought into a mirror symmetry.

6. Apparatus according to any of claims 1 to 5, wherein the combining unit is configured to perform a combination of the corneal data of the right eye and the corneal data of the left eye which comprises at least one of the following operations: a subtraction of corneal data of the right eye from the corneal data of the left eye, and vice-versa, an intersection of corneal data of the right eye and corneal data of the left eye and an addition of corneal data of the right eye and corneal data of the left eye.

7. Apparatus according to any of claims 1 to 6, further comprising:
a first mapping unit for receiving corneal data of the right eye and for generating a right eye map representing the ocular characteristics of the right eye and
a second mapping unit receiving corneal data of the left eye for generating a left eye map representing the ocular characteristics of the left eye.

8. Apparatus according to claim 7, comprising:
a further combining unit for combining the right eye map and the left eye map to generate a combined map for the patient.

9. Apparatus according to claim 8, comprising:
at least one further transformation unit for transformation of the right eye map and/or the left eye map, wherein said transformation comprises at least one of the following operations: an inversion of a map, an operation for providing symmetry between the right eye map and the left eye map, a translation of the map, a shift of the map and a rotation of the map.

10. Apparatus according to claim 9, comprising:
a further analyzing unit is configured to receive the combined map for determining further plural combined indices,
wherein the further plural combined indices can be used for diagnosis of corneal diseases.

11. Apparatus according to any of the foregoing claims, further comprising:
at least one other analysis unit is configured to receive corneal data of the right eye and corneal data of the left eye and/or the right eye map and the left eye map for determining plural indices for the right eye and plural indices for the left eye and
another combining unit for combining the plural indices for the right eye and the plural indices for the left eye to generate other plural combined indices for the patient.

12. Apparatus according to any of the foregoing claims, further comprising a comparing unit for comparing the plural combined indices with a reference, wherein the comparing unit preferably uses at least one discriminant function which comprises input variables and respective coefficients, wherein the coefficients are based on a statistical analysis of corneal data of a plurality of patients and wherein the plural combined indices and optionally the further plural combined indices and/or the other plural combined indices are taken as input data for the corresponding input variables of the discriminant functions.

13. Apparatus according to any of the foregoing claims, wherein the corneal data comprise data representing at least one of corneal hysteresis, corneal resistance factor, and other biomechanical diagnostics and/or wherein the corneal disease is at least one of keratoconus, glaucoma, or Marfan disease.

14. Apparatus according to any of the foregoing claims, wherein the input unit is configured to receive corneal data from at least one of a plurality of same-in-class measuring devices and wherein the input unit is preferably configured to transform corneal data from said at least one plurality of same-in-class measuring devices into one common data format which is used for analyzing said corneal data, wherein the same-in-class measuring devices preferably comprise corneal topographic measuring devices and/or tomographic measuring devices.

15. Method for analyzing corneal data, using an apparatus according to any one of claims 1 to 14 comprising the steps of:
receiving pre-acquired corneal data of the right eye and corneal data of the left eye of a patient, said corneal data comprising at least one of elevation corneal data, topographic corneal data, tomographic corneal data, anterior corneal data, posterior corneal data and pachymetric spatial profile data,
generating combined data for the patient from the corneal data of the right eye and the corneal data of the left eye,
determining plural combined indices from the combined data and
using said plural combined indices for diagnosis of ocular diseases, by using control data derived from a population group.

## Patentansprüche

1. Vorrichtung zum Analysieren von Hornhautdaten umfassend:
eine Eingabeeinheit, die konfiguriert ist, Hornhautdaten des rechten Auges und Hornhautdaten des linken Auges eines Patienten zu empfangen, wobei die Hornhautdaten mindestens eines von Hornhauterhöhungsdaten, topographischen Hornhautdaten, tomographischen Hornhautdaten, anterioren Hornhautdaten, posterioren Hornhautdaten und pachymetrischen räumlichen Profildaten umfassen;
eine Kombinationseinheit, die konfiguriert ist, die Hornhautdaten des rechten Auges und die Hornhautdaten des linken Auges zu empfangen, um kombinierte Daten für den Patienten zu erzeugen,
eine Analyseeinheit, die konfiguriert ist, die kombinierten Daten zum Bestimmen mehrerer kombinierter Indizes zu empfangen, wobei die Analyseeinheit konfiguriert ist, die mehreren kombinierten Indizes zur Diagnose von Hornhauterkrankungen unter Verwendung von Kontrolldaten zu verwenden, die von einer Bevölkerungsgruppe abgeleitet sind.

2. Vorrichtung nach Anspruch 1, ferner umfassend:
mindestens eine Transformationseinheit zum Transformieren der von der Eingabeeinheit empfangenen Hornhautdaten des rechten Auges und/oder der Hornhautdaten des linken Auges und zum Bereitstellen transformierter Daten an die Kombinationseinheit.

3. Vorrichtung nach Anspruch 2, wobei die Transformationseinheit konfiguriert ist, mindestens eine der folgenden Operationen auszuführen: eine Dateninversion, bevorzugt in Bezug auf eine vertikale Achse, eine Operation zum Erreichen einer Symmetrie zwischen Hornhautdaten des rechten Auges und Hornhautdaten des linken Auges, eine Datentranslation, eine Datenverschiebung und eine Datenrotation.

4. Vorrichtung nach Anspruch 2 oder 3, wobei die Transformationseinheit angepasst ist, eine Transformation von Hornhautdaten des rechten Auges und/oder von Hornhautdaten des linken Auges auszuführen, um eine beste Anpassung der Hornhautdaten des rechten Auges an die Hornhautdaten des linken Auges zu erreichen.

5. Vorrichtung nach Anspruch 4, wobei die beste Anpassung der Hornhautdaten des rechten Auges an die Hornhautdaten des linken Auges bevorzugt durch Bezugnahme auf eines der folgenden Referenzkriterien erreicht wird: a) beste Anpassung der Hornhauterhöhungsdaten des rechten Auges und der Hornhauterhöhungsdaten des linken Auges, b) dass die Hornhautdaten des rechten Auges und die Hornhautdaten des linken Auges in Bezug auf den dünnsten Punkt der Hornhaut des rechten Auges und den dünnsten Punkt der Hornhaut des linken Auges ausgerichtet sind, c) dass die Hornhautdaten des rechten Auges und die Hornhautdaten des linken Auges in Bezug auf den Hornhautscheitel der Hornhaut des rechten Auges und den Hornhautscheitel der Hornhaut des linken Auges ausgerichtet sind, d) dass die Hornhautdaten des rechten Auges und die Hornhautdaten des linken Auges in Bezug auf die Hornhautspitze der Hornhaut des rechten Auges und die Hornhautspitze der Hornhaut des linken Auges ausgerichtet sind, und e) dass die Hornhautdaten des rechten Auges und die Hornhautdaten des linken Auges in eine Spiegelsymmetrie gebracht sind.

6. Vorrichtung nach einem der Ansprüche 1 bis 5, wobei die Kombinationseinheit konfiguriert ist, eine Kombination der Hornhautdaten des rechten Auges und der Hornhautdaten des linken Auges auszuführen, die mindestens eine der folgenden Operationen umfasst: eine Subtraktion der Hornhautdaten des rechten Auges von den Hornhautdaten des linken Auges und umgekehrt, einen Schnitt von Hornhautdaten des rechten Auges und von Hornhautdaten des linken Auges und eine Addition von Hornhautdaten des rechten Auges und von Hornhautdaten des linken Auges.

7. Vorrichtung nach einem der Ansprüche 1 bis 6, ferner umfassend:
eine erste Abbildungseinheit zum Empfangen von Hornhautdaten des rechten Auges und zum Erzeugen einer rechte Augenkarte, welche die Augenmerkmale des rechten Auges darstellt, und
eine zweite Abbildungseinheit zum Empfangen von Hornhautdaten des linken Auges, um eine linke Augenkarte zu erzeugen, welche die Augenmerkmale des linken Auges darstellt.

8. Vorrichtung nach Anspruch 7, umfassend:
eine weitere Kombiniereinheit zum Kombinieren der rechten Augenkarte und der linken Augenkarte, um eine kombinierte Karte für den Patienten zu erzeugen.

9. Vorrichtung nach Anspruch 8, umfassend:
mindestens eine weitere Transformationseinheit zur Transformation der rechten Augenkarte und/oder der linken Augenkarte, wobei die Transformation mindestens eine der folgenden Operationen umfasst: eine Invertierung einer Karte, eine Operation zum Bereitstellen einer Symmetrie zwischen der rechten Augenkarte und der linken Augenkarte, eine Translation der Karte, eine Verschiebung der Karte und eine Rotation der Karte.

10. Vorrichtung nach Anspruch 9, umfassend:
eine weitere Analyseeinheit, die konfiguriert ist, die kombinierte Karte zum Bestimmen weiterer mehrerer kombinierter Indizes zu empfangen,
wobei die weiteren mehreren kombinierten Indizes zur Diagnose von Hornhauterkrankungen verwendet werden können.

11. Vorrichtung nach einem der vorstehenden Ansprüche, ferner umfassend:
mindestens eine weitere Analyseeinheit, die konfiguriert ist, Hornhautdaten des rechten Auges und Hornhautdaten des linken Auges und/oder die rechte Augenkarte und die linke Augenkarte zu empfangen, um mehrere Indizes für das rechte Auge und mehrere Indizes für das linke Auge zu bestimmen, und
eine weitere Kombinationseinheit zum Kombinieren der mehreren Indizes für das rechte Auge und der mehreren Indizes für das linke Auge, um andere mehrere kombinierte Indizes für den Patienten zu erzeugen.

12. Vorrichtung nach einem der vorstehenden Ansprüche, ferner umfassend eine Vergleichseinheit zum Vergleichen der mehreren kombinierten Indizes mit einer Referenz, wobei die Vergleichseinheit bevorzugt mindestens eine Diskriminanzfunktion verwendet, die Eingabevariablen und entsprechende Koeffizienten umfasst, wobei die Koeffizienten auf einer statistischen Analyse von Hornhautdaten mehrerer Patienten basieren, und wobei die mehreren kombinierten Indizes und optional die weiteren mehreren kombinierten Indizes und/oder die anderen mehreren kombinierten Indizes als Eingabedaten für die entsprechenden Eingabevariablen der Diskriminanzfunktionen verwendet werden.

13. Vorrichtung nach einem der vorstehenden Ansprüche, wobei die Hornhautdaten Daten umfassen, die mindestens eines von Hornhauthysterese, Hornhautresistenzfaktor und anderer biomechanischer Diagnostik darstellen, und/oder wobei die Hornhautkrankheit mindestens eine von Keratokonus, Glaukom oder Marfan-Krankheit ist.

14. Vorrichtung nach einem der vorstehenden Ansprüche, wobei die Eingabeeinheit konfiguriert ist, Hornhautdaten von mindestens einer von mehreren gleichklassigen Messvorrichtungen zu empfangen, und wobei die Eingabeeinheit bevorzugt konfiguriert ist, Hornhautdaten von der mindestens einen Vielzahl von gleichklassigen Messvorrichtungen in ein gemeinsames Datenformat zu transformieren, das zum Analysieren der Hornhautdaten verwendet wird, wobei die gleichklassigen Messvorrichtungen bevorzugt topographische und/oder tomographische Hornhautmessvorrichtungen umfassen.

15. Verfahren zum Analysieren von Hornhautdaten unter Verwendung einer Vorrichtung nach einem der Ansprüche 1 bis 14, umfassend die Schritte:
Empfangen von zuvor erfassten Hornhautdaten des rechten Auges und Hornhautdaten des linken Auges eines Patienten, wobei die Hornhautdaten mindestens eines von Hornhauterhöhungsdaten, topographischen Hornhautdaten, tomographischen Hornhautdaten, anterioren Hornhautdaten, posterioren Hornhautdaten und pachymetrischen räumlichen Profildaten umfassen,
Erzeugen kombinierter Daten für den Patienten von den Hornhautdaten des rechten Auges und den Hornhautdaten des linken Auges,
Bestimmen mehrerer kombinierter Indizes von den kombinierten Daten und
Verwenden mehrerer kombinierter Indizes für die Diagnose von Augenkrankheiten durch Verwenden von Kontrolldaten, die von einer Bevölkerungsgruppe abgeleitet wurden.

## Revendications

1. Appareil pour l'analyse de données cornéennes comprenant :
une unité d'entrée configurée pour recevoir des données cornéennes de l'œil droit et des données cornéennes de l'œil gauche d'un patient, lesdites données cornéennes comprenant au moins une parmi des données cornéennes d'élévation, des données cornéennes topographiques, des données cornéennes tomographiques, des données cornéennes antérieures, des données cornéennes postérieures et des données de profil spatiales pachymétriques ;
une unité combinée configurée pour recevoir les données cornéennes de l'œil droit et les données cornéennes de l'œil gauche pour la génération de données combinées pour le patient,
une unité d'analyse configurée pour recevoir les données combinées pour la détermination d'une pluralité d'indices combinés, dans laquelle l'unité d'analyse est configurée pour utiliser ladite pluralité d'indices combinés pour un diagnostic de maladies cornéennes à l'aide de données de contrôle dérivées d'un groupe de population.

2. Appareil selon la revendication 1, comprenant en outre :
au moins une unité de transformation pour une transformation des données cornéennes de l'œil droit et/ ou des données cornéennes de l'œil gauche reçues à partir de ladite unité d'entrée, et pour la fourniture de données transformées à l'unité de combinaison.

3. Appareil selon la revendication 2, dans lequel ladite unité de transformation est configurée pour exécuter au moins une parmi les opérations suivantes, une inversion de données, de préférence par rapport à un axe vertical, une opération afin d'obtenir une symétrie entre des données cornéennes de l'œil droit et des données cornéennes de l'œil gauche, une translation de données, un changement de données et une rotation de données.

4. Appareil selon la revendication 2 ou 3, dans lequel l'unité de transformation est adaptée pour exécuter une transformation de données cornéennes de l'œil droit et/ ou de données cornéennes de l'œil gauche afin d'obtenir un meilleur ajustement des données cornéennes de l'œil droit avec les données cornéennes de l'œil gauche.

5. Appareil selon la revendication 4, dans lequel le meilleur ajustement des données cornéennes de l'œil droit avec les données cornéennes de l'œil gauche est obtenue de préférence par la référence à l'un des critères de référence suivants : a) meilleur ajustement de l'élévation des données cornéennes de l'œil droit et de l'élévation des données cornéennes de l'œil gauche, b) que les données cornéennes de l'œil droit et les données cornéennes de l'œil gauche sont alignées par rapport au point le plus fin de la cornée de l'œil droit et au point le plus fin de la cornée de l'œil gauche, c) que les données cornéennes de l'œil droit et les données cornéennes de l'œil gauche sont alignées par rapport au sommet cornéen de la cornée de l'œil droit et au sommet cornéen de la cornée de l'œil gauche, d) que les données cornéennes de l'œil droit et les données cornéennes de l'œil gauche sont alignées par rapport à l'apex cornéen de la cornée de l'œil droit et à l'apex cornéen de la cornée de l'œil gauche, et e) que les données cornéennes de l'œil droit et les données cornéennes de l'œil gauche sont amenés dans une symétrie miroir.

6. Appareil selon l'une quelconque des revendications 1 à 5, dans lequel l'unité de combinaison est configurée pour exécuter une combinaison des données cornéennes de l'œil droit et des données cornéennes de l'œil gauche, qui comprend au moins une parmi les opérations suivantes : une soustraction de données cornéennes de l'œil droit à partir des données cornéennes de l'œil gauche, et vice-versa, une intersection de données cornéennes de l'œil droit et de données cornéennes de l'œil gauche et une addition de données cornéennes de l'œil droit et de données cornéennes de l'œil gauche.

7. Appareil selon l'une quelconque des revendications 1 à 6, comprenant en outre :
une première unité de cartographie pour la réception de données cornéennes de l'œil droit et pour la génération d'une carte de l'œil droit représentant les caractéristiques oculaires de l'œil droit et
une deuxième unité de cartographie recevant des données cornéennes de l'œil gauche pour la génération d'une carte de l'œil gauche représentant les caractéristiques oculaires de l'œil gauche.

8. Appareil selon la revendication 7, comprenant :
une unité de combinaison supplémentaire pour la combinaison de la carte de l'œil droit et de la carte de l'œil gauche afin de générer une carte combinée pour le patient.

9. Appareil selon la revendication 8, comprenant :
au moins une unité de transformation supplémentaire pour une transformation de la carte de l'œil droit et/ ou de la carte de l'œil gauche, dans laquelle ladite transformation comprend au moins une parmi les opérations suivantes : une inversion d'une carte, une opération pour la fourniture d'une symétrie entre la carte de l'œil droit et la carte de l'œil gauche, une translation de la carte, un changement de la carte et une rotation de la carte.

10. Appareil selon la revendication 9, comprenant :
une unité d'analyse supplémentaire est configurée pour recevoir la carte combinée pour la détermination d'une pluralité d'indices combinés supplémentaires,
dans lequel la pluralité d'indices combinés supplémentaires peut être utilisée pour un diagnostic de maladies cornéennes.

11. Appareil selon l'une quelconque des revendications précédentes, comprenant en outre :
au moins une autre unité d'analyse est configurée pour recevoir des données cornéennes de l'œil droit et des données cornéennes de l'œil gauche et/ ou la carte de l'œil droit et la carte de l'œil gauche pour la détermination de la pluralité d'indices pour l'œil droit et de la pluralité d'indices pour l'œil gauche et
une autre unité de combinaison pour la combinaison de la pluralité d'indices pour l'œil droit et de la pluralité d'indices pour l'œil gauche afin de générer une autre pluralité d'indices combinés pour le patient.

12. Appareil selon l'une quelconque des revendications précédentes, comprenant en outre une unité pour la comparaison de la pluralité d'indices combinés avec une référence, dans lequel l'unité de comparaison utilise de préférence au moins une fonction discriminante qui comprend des variables d'entrée et des coefficients respectifs, dans lequel les coefficients sont basés sur une analyse statistique de données cornéennes d'une pluralité de patients et dans lequel la pluralité d'indices combinés et éventuellement la pluralité d'indices combinés supplémentaires et/ ou l'autre pluralité d'indices combinés sont considérées comme des données d'entrée pour les variables d'entrée correspondantes des fonctions discriminantes.

13. Appareil selon l'une quelconque des revendications précédentes, dans lequel les données cornéennes comprennent des données représentant au moins un parmi l'hystérésis cornéenne, le facteur de résistance cornéenne, et d'autres diagnostics biomécaniques et/ ou dans lequel la maladie cornéenne est au moins un parmi le kératocus, le glaucome ou la maladie de Marfan.

14. Appareil selon l'une quelconque des revendications précédentes, dans lequel l'unité d'entrée est configurée pour recevoir des données cornéennes à partir d'au moins une de la pluralité de dispositifs de mesure de même classe et dans lequel l'unité d'entrée est de préférence configurée pour transformer des données cornéennes à partir de ladite au moins une pluralité de dispositifs de mesure de même classe dans un format de données commun qui est utilisé pour l'analyse desdites données cornéennes, dans lequel les dispositifs de mesure de même classe comprennent de préférence des dispositifs de mesure topographique cornéenne et/ ou des dispositifs de mesure tomographique.

15. Procédé pour l'analyse de données cornéennes, à l'aide d'un appareil selon l'une quelconque des revendications 1 à 14, comprenant les étapes de :
la réception de données cornéennes pré-acquises de l'œil droit et de données cornéennes de l'œil gauche d'un patient, lesdites données cornéennes comprenant au moins une parmi des données cornéennes d'élévation, des données cornéennes topographiques, des données cornéennes tomographiques, des données cornéennes antérieures, des données cornéennes postérieures et des données de profil spatiales pachymétriques,
la génération de données combinées pour le patient à partir des données cornéennes de l'œil droit et des données cornéennes de l'œil gauche,
la détermination d'une pluralité d'indices combinés à partir des données combinées et
à l'aide de ladite pluralité d'indices combinés pour un diagnostic de maladies oculaires, en utilisant des données de contrôle dérivées d'un groupe de population.
